# EUROPEAN PATENT SPECIFICATION

(11) **EP 0 638 103 B1**
(45) Date of publication and mention of the grant of the patent: **08.09.1999**
(21) Application number: 94907636.8
(22) Date of filing: 25.02.1994
(51) Int. Cl.: C08G 65/00, C08G 65/32, C07C 43/12

(54) **PREPARATION OF FLUORINATED POLYETHERS**
HERSTELLUNG VON FLUORIERTEN POLYETHERN
PEPARATION DE POLYETHERS FLUORES

(30) Priority: 26.02.1993 GB 9303916; 26.02.1993 GB 9303917
(43) Date of publication of application: 15.02.1995
(73) Proprietor: F2 Chemicals Limited, Preston, Lancashire PR4 0XJ (GB)
(72) Inventor: CHAMBERS, Richard Dickinson, South Road, Durham DH1 3LE (GB); JOEL, Andrew Keith, Salwick, Preston PR4 0XJ (GB)
(74) Representative: Pawlyn, Anthony Neil
(86) International application number: GB9400381
(87) International publication number: WO9419391

(56) References cited:
- EP-A- 0 247 887
- EP-A- 0 269 029
- EP-A- 0 344 935
- WO-A-90/03353
- US-A- 3 897 502

## Description

The present invention relates to a process for the preparation of a perfluoropolyether.

Various perfluoropolyethers (that is, fully fluorinated polymeric ether compounds) have been developed in recent years and are available as commercial products. These products display a variety of useful properties, including high resistance to thermal and chemical attack, very low solubility in most usual solvents, and very good lubricating properties.

In EP0247887A by R D Chambers there is described a route to such polyethers of general formula (1) as follows: 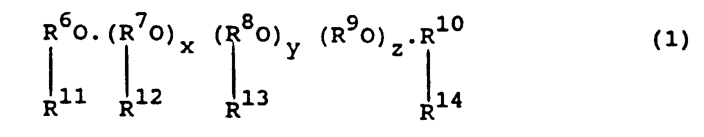 wherein the symbols R⁶ to R¹⁴ each represents a fully fluorinated hydrocarbon group, x is an integer and each of y and z is zero or an integer, and the sum of x, y and z is at least 2 and not greater than 100, preferably not greater than 50. Preferably the ratio of (x+y):z is as high as possible. Each of the groups R⁶, R⁷, R⁸ and R¹⁰ may contain a substituent fully fluorinated hydrocarbon group in addition to the groups R¹¹, R¹², R¹³ and/or R¹⁴.

The polyethers (1) are prepared by direct fluorination with elemental fluorine of intermediate substituted polyethers of general formula (2) as follows: 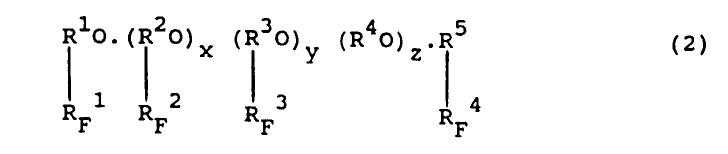 wherein the symbols R¹, R², R³, R⁴ and R⁵ each represent substituted or unsubstituted hydrocarbon groups, which groups may differ, the symbols R_{F}¹, R_{F}², R_{F}³ and R_{F}⁴ each represent fluorine-containing hydrocarbon groups containing at least two carbon atoms and at least two fluorine atoms, which groups may differ, or the combination R¹-R_{F}¹ and/or the combination R₅-R_{F}⁴ represents a fluorine-containing alkyl or alkenyl group, x is an integer and each of y and z is zero or an integer, and the sum of x, y and z is at least 2 and not greater than 100, preferably not greater than 50. Within these limits, the ratio of (x+y):z is preferably as high as possible.

In the final product, not only the hydrogen atoms on the main polyether chain but also those remaining in the groups R_{F}¹ to R_{F}⁴ may be replaced by fluorine.

The prior art method of carrying out the said fluorination as described in EP0247887A is not ideal and the purpose of the present invention is to provide an improved method.

According to the present invention there is provided a process for the preparation of a perfluoropolyether having a general formula: 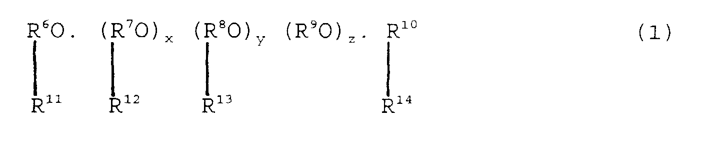 as defined hereinbefore, which process includes the direct fluorination with elemental fluorine of a polyether having a general formula: 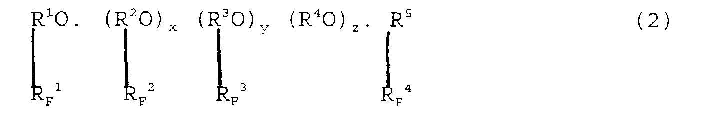 as defined hereinbefore, or cyclic polyethers such as crown ethers, or polyethers selected from diethyl (polyethylene oxides); dihydroxy (polyethylene oxides); diethyl (polytetrahydrofurans) and cyclic polyethers, characterised in that the fluorination is carried out in the presence of a solvent which is an ether having at least one substituent R_{F}, wherein R_{F} is a fluorinated hydrocarbon group, the fluorination of the polyether proceeding in concert with the fluorination of the solvent.

The said solvent ether is preferably a fluorinated volatile ether.

The said solvent ether preferably has at least two groups R_{F} which may be the same or different groups.

Each group R_{F} preferably has from two to four carbon atoms and at least two fluorine atoms.

The said solvent ether may be an alicyclic ether having from 3 to 5 carbon atoms in its alicyclic ring. Preferably the said solvent ether is of the formula:

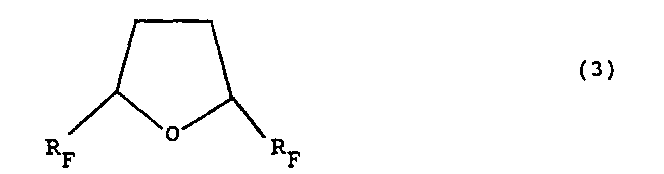

Preferably in formula (3) each R_{F} group is CF₂CFHCF₃.

The inventor has found that fluorinated ethers can be efficient solvents for the process according to the first aspect. As the fluorination of the polyethers of formula (2) proceeds, it does so in concert with fluorination of the solvent, keeping the system mobile. For example, at the end of the reaction there is a perfluorinated solvent as well as the desired perfluoropolyether and, if the solvent is volatile, this assists separation of the product from the solvent.

Although the fluorination of the intermediate substituted polyethers (2) can be begun at room temperature, it is desirable that steps be taken nonetheless to control the heat generated by the reaction. Such steps, which depend upon balancing the rate of addition of fluorinating agent against the rate of removal of heat, are well known to those skilled in the art and need not be described at length herein. They include carrying out the reaction in a thin film reactor, wherein the reaction mixture is spread in a thin film over a cooled surface of good thermal conductivity. Such reactors include the Bigelow type reactor. An alternative method of controlling the heat of reaction is by evaporative heat transfer using a refluxing volatile liquid diluent. A third method lies in the use of a so-called "aerosol" reactor.

In one particularly preferred form of the invention, fluorination is effected by introducing a gaseous mixture of fluorine and nitrogen into the substituted polyether (2) in liquid form. The gaseous mixture may be introduced as a stream of fine bubbles, preferably in a reactor wherein the polyether and the gaseous mixture meet in counterflow. The reaction is preferably carried out at atmospheric pressure or more preferably under a somewhat elevated pressure. If desired, the reaction may be assisted by, for example, irradiating the reaction mixture with ultra-violet light.

When the fluorinating agent is a mixture of fluorine and nitrogen, the concentration of fluorine in the mixture may be progressively increased as the reaction proceeds until the polyether (2) is fully fluorinated. For example, the reaction may be begun using a fluorine/nitrogen mixture containing a relatively small percentage, say 5 to 25 per cent, of fluorine, which may progressively be increased to 100 per cent fluorine to remove the last unsubstituted hydrogen atoms from the polyether molecule.

According to the present invention in a preferred form there is the process for the preparation of intermediates having units of general formula (2) hereinbefore which process includes the step of reacting a linear or branched chained or cyclic polyether of or having units of general formula (4) or (5) given hereafter with a straight chained or cyclic fluoroalkene containing at least two fluorine atoms in the presence of a sensitising medium which when energised helps to induce the reaction, ultra-violet radiation being applied to the said medium to provide energisation of the medium.

Formulae (4) and (5) are as follows: 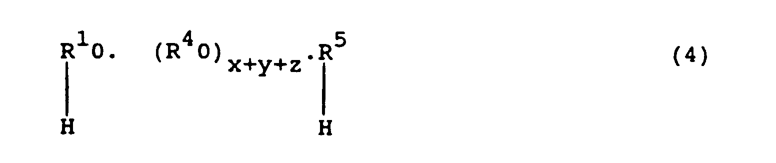 or

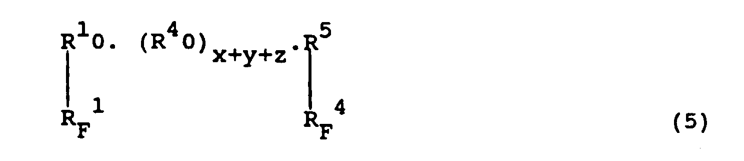

In the foregoing general formulae (4) and (5), the groups R¹ and R⁵ may each contain a single carbon atom but preferably at least the group R⁴ contains at least 2 carbon atoms, more preferably from 2 to 4 carbon atoms. The groups may be linear, branched or cyclic and adjacent groups may be combined with the intervening oxygen atom in a cyclic ether group. The group R⁴ may represent a mixture of groups.

Thus one or more of the groups R¹, R⁴ and R⁵ may be a -C₂H₄- group or a -C₃H₆- or -C₄H₈- group or the polyether (4) or (5) may be a poly(cyclic ether). If desired, the end-groups R¹ and R⁵ in particular may be substituted groups, for example -C₂H₄- groups in which at least one hydrogen atom has already been substituted by a fluorine atom.

The said sensitising medium is an organic liquid which is desirably a solvent for the reagents having a strong extinction coefficient in the spectral region of the applied ultra-violet radiation and may, for example, be acetone or trifluorethanol.

Examples of polyethers which may be employed as the compound of Formula (2) in the process according to the preferred form of the present invention include diethyl (polyethylene oxides); dihydroxy (polyethylene oxides); diethyl (poly tetrahydrofurans) and cyclic polyethers such as crown ethers.

Examples of fluoroalkenes which may be employed in the process according to the preferred form of the present invention include fluoropropenes, fluorobutenes, fluoropentenes and fluorohexenes containing at least five fluorine atoms, eg pentafluoropropene, hexafluoropropene and octafluorobutene and analogous ring containing compounds, eg fluorocyclobutenes fluorocyclopentenes and fluorocyclohexenes containing at least six fluorine atoms.

Beneficially and unexpectedly the process of the preferred form of the present invention allows a greater number of groups in the intermediates of formula (2) to be obtained as fluorinated groups than by using the aforementioned prior art processes for the preparation of such intermediates. The possibility of incorporating a greater number of fluorinated groups is beneficial because it allows greater choice of properties in the resultant perfluorinated end products.

Comparative examples illustrating this improvement are shown in Table 1 as follows:

**Table 1**

| **DEPEG** | **MAX** | **DTBP** | **gamma-rays** | **UV** |
|---|---|---|---|---|
| 400 | 11 | 6 | - | 8 |
| 600 | 15 | 11*, 13* | 8 | 14 |
| 2000 | 45 | 20 | 17 | 35 |

| | | | | |
|---|---|---|---|---|
| * different examples of the same route | | | | |

In each case in Table 1 a fluorinated polyether compound of formula (2) is produced from an unfluorinated diethyl (polyethylene glycol) alternatively known as diethyl (polyethylene oxide) and hexafluoropropene as the fluoroalkene by three different routes. The diethyl polyethylene glycol in each case is indicated under the heading "PEG" where the number stated is the average molecular weight of the commercially available polyethylene glycol used to form the diethyl derivative. The numbers under the column heading "MAX" are the maximum number of groups in the diethyl polyethylene glycol molecule which are available to be converted to fluoro-groups. The numbers under the other headings are the number of fluoro-groups produced (or fluorine incorporations) for each diethyl polyethylene glycol using the route in question. The first route denoted by the column heading "DTBP" is the prior art route of Examples 2, 4, 6 and 8 in EP 0247887 using ditertiary butyl peroxide (DTBP). The second route denoted by the column heading "gamma-rays" is another prior art route mentioned as a possibility but not exemplified in EP 0247887. This route as used in this comparative example is described in Procedure E and Examples A1 and A2 below.

The third route denoted by the column heading "UV" is the process of the preferred form of the present invention as exemplified in Examples 7 to 9 given below.

Further benefits are obtained by using the process of the preferred form of the present invention as follows. A cleaner product is obtained than by using the DTBP since the latter results in peroxide residues. Product breakdown experienced with the DTBP route and the use of relatively large quantities of unpleasant peroxides is avoided. The process according to the present invention gives results which are more reproducible than those obtained with the prior art. The process according to the present invention may be carried out at temperatures lower than those required in the DTBP route. The latter requires temperatures of typically 140°C whereas the process of the present invention can be carried out at temperatures of 50°C or below, eg room temperature eg 20°C to 25°C. The "gamma-rays" route also requires elevated temperatures to obtain satisfactory reaction.

Furthermore, the process according to the preferred form of the present invention allows less reactive polyethers used as starting materials to be fluorinated more readily to compounds of Formula (2) compared with the prior art routes. For example, trioxan which has a very low reactivity and crown ethers are easier to fluorinate by the process of the present invention.

A further unexpected benefit of the preferred form of the process according to the present invention is that if the fluoralkene used in the process is or includes a cyclic fluoralkene cyclic fluorinated groups may be introduced much more extensively in the intermediates of formula (2) than by using the prior art processes. The advantage in this improvement is that the presence of cyclic groups in the intermediates obtained thereby gives greater opportunities to tailor the perfluorinated end products of formula (1) to end user requirements. For example, perfluorocyclobutene of formula (6) and perfluorcyclopentene of formula (7) as follows give as side groups in the compounds of formula (2) the following cyclic fluorinated groups of formulae (6a) and (7a) as follows:

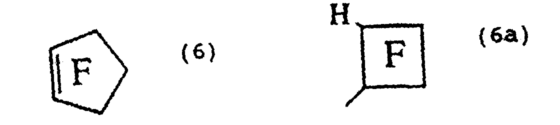

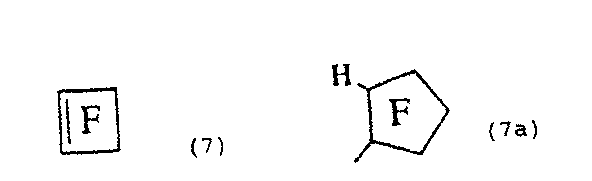

In the process according to the preferred form of the present invention the ultraviolet (UV) radiation employed may be obtained from any known UV source conventionally used in photochemistry. For example it may be obtained from a mercury lamp. The radiation source may have a power output of 0.1 KW to 10 KW. The reaction vessel may be located a distance of 50mm to 200mm from the radiation source.

Embodiments of the present invention will now be described, by way of example only, with reference to the following Examples:

### Example 1. Fluorination of poly (2H-hexafluoropropyl)-[diethyl poly (ethylene glycol) 600]

Poly (2H-hexafluoropropyl)-[diethyl poly (ethylene glycol) 600] of formula (8) as follows: 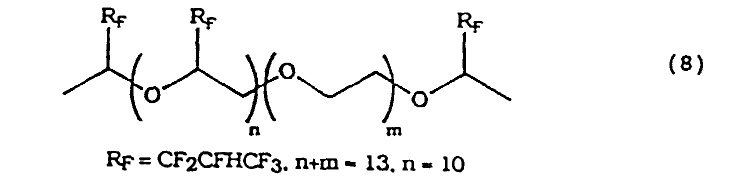 (where the average number of ethylene glycol units is about 13, and the average number of 2H-hexafluoropropyl groups is 12) (2.2g) was dissolved in 1,1,1,2,3,3-hexafluoro-4,5-di(l'-methyl-2',2',3',4',4'-,4'-hexafluorobutoxy)pentane of formula (9) as follows: 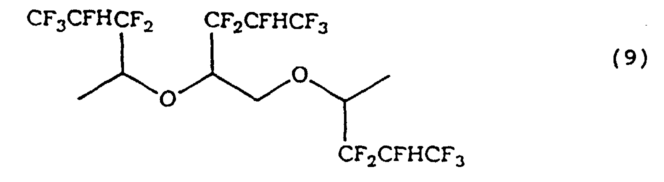 (7.8g) and the solution placed in a fluorinated ethylene propylene (FEP) tube of internal diameter approximately 4.5mm. Fluorine, diluted in nitrogen, was introduced into the FEP tubing through a polytetrafluoroethylene (PTFE) capillary tube, at a rate that would provide a steady stream of bubbles over 18 days. In each day an 800 ml cylinder, originally containing 10 atm of gas, was discharged through the reaction vessel. The concentration of fluorine in nitrogen was increased over this period as follows: 5 per cent (4 days); 10 per cent (2 days); 15 per cent, 20 per cent, 25 per cent, 30 per cent and 50 per cent (1 day each). For the remaining 7 days 50 per cent fluorine in nitrogen was used, whilst concurrently irradiating the reaction mixture with ultra-violet light from a 1000 watt, medium pressure mercury lamp at a distance of 0.1m, the reaction mixture also being cooled by a fan to approximately 50°C.

After complete fluorination had been achieved, as shown by ¹H n.m.r. spectroscopy, the solvent was removed under vacuum, having also been perfluorinated to perfluoro[1,2-di(1'-methylbutoxy)]pentane of formula (10) as follows: 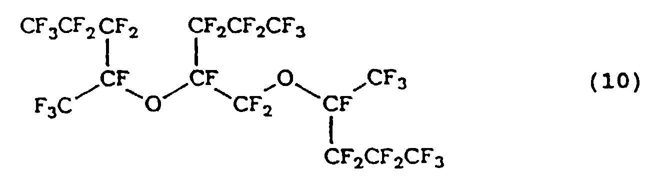 (6.9g). A fairly viscous, clear, colourless liquid was left, which ¹⁹F n.m.r. spectroscopy showed to be the desired perfluoropolyether of formula (11) as follows: 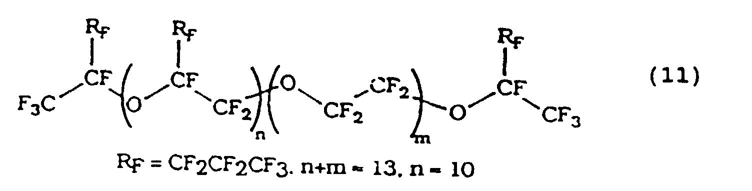 in a yield of 1.3g.

### Example 2. Fluorination of poly(2H-hexafluoropropyl)-[diethyl poly (ethylene glycol) 600]

Poly(2H-hexafluoropropyl)-[diethyl poly (ethylene glycol) 600] of formula (8) as defined hereinbefore (4.7g) was dissolved in 2,5-di(2H-hexafluoropropyl)oxolane of formula (12) as follows: 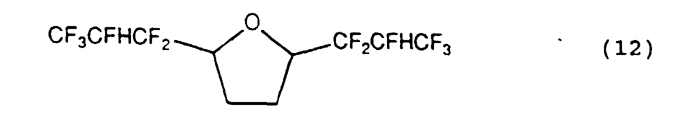 (10ml) in a length of FEP tubing. Fluorine in nitrogen was passed through the solution, the concentration of fluorine in nitrogen being increased stagewise as follows: 5 per cent; 10 per cent; 20 per cent; 30 per cent and 50 per cent, with at each stage 7 litres of the gas mixture being passed through in 8 to 12 hours. A further 3 stages were required of 50 per cent fluorine in nitrogen under ultra-violet light irradiation as carried out in Example 1. The perfluorinated solvent of formula (13) as follows: 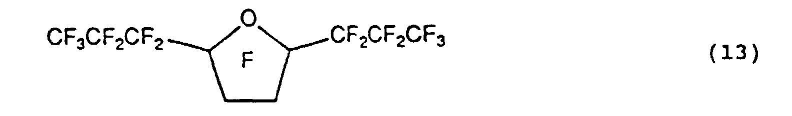 was removed under vacuum (5.2g), the loss of mass being due to the volatility of the perfluorinated material. A perfluoropolyether of formula (11) as hereinbefore defined (3.7g) remained as a viscous, clear, colourless, liquid.

### Example 3. Fluorination of poly(2H-hexafluoropropyl)-[diethyl poly(ethylene glycol) 600]

Poly (2H-hexafluoropropyl)-[diethyl poly(ethylene glycol) 600] of formula (8) as defined hereinbefore (7.6 g) was dissolved in 2,5-di(2H-hexafluoropropyl)oxolane of formula (12) as defined hereinbefore (11.0 g) and placed in an FEP tube. Fluorine in nitrogen was bubbled through the solution over a period of 18 days, the concentration of fluorine in nitrogen being increased stagewise as described in relation to Example 1.

After complete fluorination had been achieved, as shown by ¹H n.m.r. spetroscopy, the solvent was removed under reduced pressure, having also been perfluorinated to perfluoro-2,5-dipropyloxolane of formula (13) as defined hereinbefore. A clear, colourless liquid remained, which ¹⁹F n.m.r. showed to be a perfluoropolyether of formula (11) as defined hereinbefore, in a yield of 6.0g, 54%.

### Example 4. Fluorination of poly(2H-hexafluoropropyl)-[diethyl poly(ethylene glycol) 400]

Poly (2H-hexafluoropropyl)-[diethyl poly(ethylene glycol) 400] of formula (14) as follows: 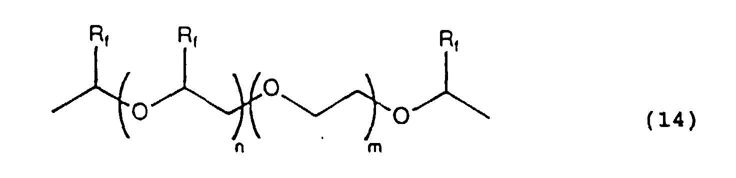 where R_{f} = CF₂CFHCF₃ n_6, m_2
3.6g (where the average number of ethylene glycol units is about 8, and the average number of 2H-hexafluoropropyl groups is 8) was dissolved in 2,5-di(2H-hexafluoropropyl)oxolane of formula (12) as defined hereinbefore (2.5g) and placed in an FEP tube. Fluorine in nitrogen was bubbled through the solution, as hereinbefore described, over a period of 22 days, the concentration of fluorine in nitrogen being increased stagewise as follows: 5 per cent (6 days); 10 per cent, 15 per cent, 20 per cent, 25 per cent, 30 per cent and 50 per cent (2 days each). For the remaining 4 days, 50 per cent fluorine in nitrogen was used, whilst concurrently irradiating the reaction mixture with ultra-violet light as hereinbefore described.

After complete fluorination had been achieved, the solvent was removed under reduced pressure, having also been perfluorinated to perfluoro-2,5-dipropyloxolane of formula (13) as defined hereinbefore. The remaining liquid was distilled at 200°C/0.05 mm Hg on a Kugelrohr apparatus to give a clear, colourless liquid which ¹⁹F n.m.r. spectroscopy showed to be the desired perfluoropolyether of formula (15) as follows: 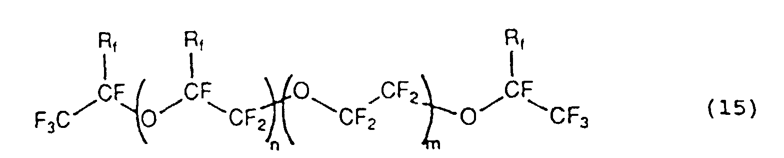 where R_{f} = CF₂CF₂CF₃ n≈6, m≈2
in a yield of 5.4g, 64%.

### Example 5. Fluorination of poly(2H-hexafluoropropyl)-diethyl polytetrahydrofuran

Poly (2H-hexafluoropropyl)-diethyl polytetrahydrofuran of formula (16) as follows: 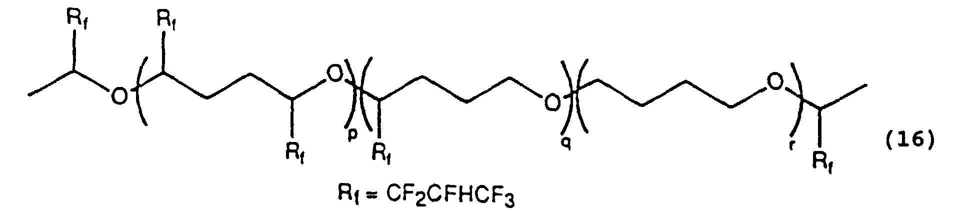 (6.9g) was dissolved in 2,5-di(2H-hexafluoropropyl)oxolane of formula (12) as defined hereinbefore (12.4g) and placed in an FEP tube. Fluorine in nitrogen was bubbled through the solution over a period of 22 days, the concentration of fluorine in nitrogen being increased stagewise as described in relation to Example 4.

After complete fluorination had been achieved as shown by ¹H n.m.r. spectroscopy, the solvent was removed under reduced pressure, having also been perfluorinated to perfluoro-2,5-dipropyloxolane of formula (13) as defined hereinbefore. The remaining liquid was distilled at 200°C/0.05 mmHg using a Kugelrohr apparatus to give a clear, colourless liquid which ¹⁹F n.m.r. showed to be the desired perfluoropolyether of formula (17) as follows: 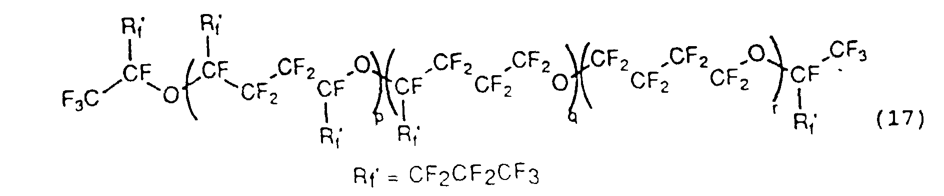 in a yield of 5.4g, 64%.

### Example 6. Fluorination of poly(2H-hexafluoropropyl)-18-crown-6

6.9g of poly(2H-hexafluoropropyl)-18-crown-6 of formula (18) as follows: 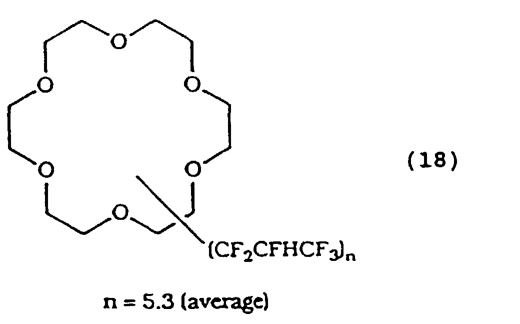 (where the average number of 2H-hexafluoropropyl groups is 5.3) was dissolved in 2,5-di(2H-hexafluoropropyl)oxolane of formula (12) as hereinbefore described (12.4g) in a length of FEP tubing. A mixture of fluorine in nitrogen was introduced into the tubing and the concentration of fluorine in the mixture was increased over a period of 15 days in a first stage as follows: 5 per cent (6 days); 10 per cent (2 days); 15 per cent, 20 per cent, 25 per cent and 30 per cent (1 day each), and 50 per cent (3 days). 50 per cent fluorine in nitrogen was then used in a second stage whilst the reaction mixture was irradiated with ultra-violet light, as described in relation to Example 1, for 4 days. The resulting perfluorinated solvent of formula (13) as hereinbefore described was removed under vacuum to yield a clear, colourless material, the perfluorinated product of formula (19) as follows: 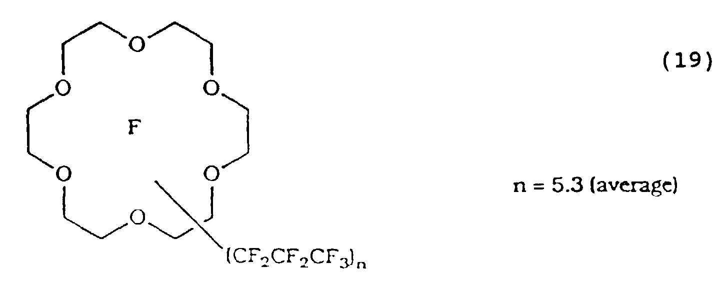 (7.3g), which could be distilled on a Kugelruhr apparatus at about 150°/0.03 mmHg.

An advantage of the present invention is that it overcomes the problem of maintaining a mobile medium for the fluorination steps in the route for producing perfluorinated polyethers. Previously, use of highly fluorinated solvent was not possible because such solvents were unable to dissolve sufficient quantities of starting materials to make fluorination practicable and, in addition, this limited the ability to control the fluorination steps.

As already indicated, perfluoropolyethers are distinguished by an attractive combination of properties including high resistance to thermal and chemical attack, very low solubility in many solvents and excellent lubricating properties. They are therefore of particular value as lubricants in chemically aggressive environments (for example in oxygen and chlorine compressors) or for highly sensitive equipment (for example the drives for computer disks and tapes) or as surface lubricants for computer disks and tapes. Perfluoropolyethers may also be sprayed on to porous surfaces (for example masonry) to provide repellency to liquid water (for example in the form of acid rain). They have further value as highly stable hydraulic fluids.

Examples of the preparation of the intermediate compounds of formula (2) using processes of the preferred form of the present invention using ultra violet radiation will now be described in the following Examples.

In the Examples which follow, the following general procedure Procedure A was used to react the starting polyether of formula (4) or (5) with hexafluoropropene (or alternative fluoroalkene).

### Procedure A

Solid and liquid reagents were introduced into a glass Carius tube (of capacity about 60 ml), together with solvent if used, and degassed twice by freeze-thawing. Hexafluoropropene (HFP) was then introduced into the cooled (by liquid air) Carius tube using standard vacuum line techniques and the tube was sealed. After regaining room temperature, the Carius tube was irradiated with UV radiation (approximately 1000W from a medium pressure mercury lamp at a distance of about 100 mm), whilst being cooled by an electric fan to a temperature of about 60°C. Subsequently, the tube was cooled (by liquid air) and opened. Any remaining HFP was recovered as the tube returned to room temperature.

### Example 7 : Fluorination of diethylpoly(ethylene oxide) derived from poly(ethylene oxide) of average molecular weight 400 - (herein called "DEPEG 400")

A Carius tube was charged with DEPEG 400 (4.4g, 9.8 mmol), acetone (11.9g) and HFP (18.8g, 125 mmol), and irradiated with UV radiation for 3 days all in the manner of Procedure A. HFP (4.9 g) was recovered and acetone removed under reduced pressure, to leave an almost colourless, viscous liquid, poly(2H-hexafluoropropyl)-a -ethyl-w-ethoxy poly(ethylene oxide) 400 which is represented by formula (8) as follows, with 8 equivalents of HFP incorporated (10.3 g, 63.8%). The IR number 3; and NMR data number 4, found for this product are consistent with the structure of formula (8) wherein n=6 and m=2. 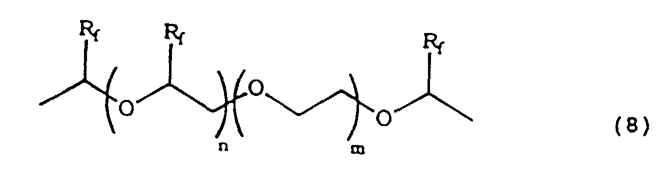 DEPEG 400 used in the above Examples was itself prepared in the following way, Procedure B.

### Procedure B

Sodium hydride (8.3 g, 207 mmol, 60% dispersion in oil) was washed in petroleum ether (3 x 100 ml), residual petroleum ether being removed under vacuum. Dried (Dean-Stark) poly(ethylene oxide) 400 (PEG 400) (32.7 g, 82 mmol) in toluene (100 ml) was added dropwise and the reaction stirred mechanically, under dry nitrogen, for 5 hours. Diethyl sulphate (15.7 g, 102 mmol) was then added dropwise, and stirring continued a further 18 hours. Solvent and remaining diethyl sulphate were removed under reduced pressure. Dichloromethane (80 ml) was added to the reaction mixture, which was then passed down a column of aluminium oxide (approximately 300 g) in dichloromethane. The solution in dichloromethane obtained was dried with magnesium sulphate. Rigorous removal of the dichloromethane gave a viscous, clear liquid, DEPEG 400 (27.1 g, 73.7%); NMR number was 2: IR analysis showed that no hydroxy groups were present.

### Example 8: Fluorination of diethylpoly(ethylene oxide) derived from poly(ethylene oxide) of average molecular weight 600 - herein called "DEPEG 600"

A Carius tube was charged with DEPEG 600 (4.0 g), acetone (7.9 g) and HFP (12.9 g), and irradiated with UV radiation for 3 days all in the manner of Procedure A. HFP (0.1 g) was recovered and acetone removed under reduced pressure, to leave an almost colourless, viscous liquid, poly(2H-hexafluoropropyl)-α -ethyl-w-ethoxy-poly(ethylene oxide) 600 with 14 equivalents of HFP incorporated (2.3 g, 65.0%). IR and NMR data measured were consistent with the structure of formula (8) wherein n=12 and m=1.

The DEPEG 600 used in Example 8 was produced by Procedure C as follows.

### Procedure C

Using the method of Procedure B above a dried solution of poly(ethylene oxide) 600 (PEG 600) (80.4 g, 134 mmol) in toluene (120 ml) was dripped onto washed sodium hydride (11.6 g of 60% dispersion in oil, 290 mmol) and stirred (5 hours). Diethyl sulphate (22.0 g, 143 mmol) was subsequently added and stirring continued (8 hours). After removal of the volatiles, passing down an alumina column in dichloromethane, drying and removal of a solvent a white, waxy solid was obtained, DEPEG 600 (69.2 g, 79.4%); IR and NMR data were consistent with those for the lower molecular weight material obtained in Procedure B; IR analysis showed that no hydroxy groups were present.

### Example 9 - Fluorination of diethyl poly(ethylene oxide) derived from poly(ethylene oxide) of average molecular weight 2000 - herein called DEPEG 2000)

A Carius tube was charged with DEPEG 2000 (5.3 g, 2.6 mmol), acetone (11.9 g) and HFP (18.3 g, 122 mmol), and was irradiated with UV radiation for 3 days all in the manner of Procedure A. HFP (4.5 g) was recovered and acetone removed under reduced pressure, to leave an almost colourless, viscous liquid, poly(2H-hexafluoropropyl)-α -ethyl-w-ethoxy poly(ethylene oxide) 2000 with 35 equivalents of HFP incorporated (12.8 g, 67.8%); IR and NMR data measured were consistent with the structure of formula (8) wherein n=33 and m=10.

DEPEG 2000 used in Example 9 was produced by Procedure D as follows.

### Procedure D

Using the method of Procedure B above a dried solution of poly(ethylene oxide) 2000 (PEG 2000) (25.2 g, 12.6 mmol) in toluene (100 ml) was dripped on to washed sodium hydride (1.4 g of 60% dispersion in oil, 35 mmol) and stirred (5 hours). Diethyl sulphate (2.3 g, 15 mmol) was subsequently added and stirring continued (8 hours). Volatile components were removed under vacuum, and the residue passed down an alumina column in dichloromethane. After drying and removal of solvent a white solid was obtained, DEPEG 2000 (19.4 g, 75.3%). IR and NMR data were consistent with those for the analogous lower molecular weight materials obtained in Procedures B and C described above. IR analysis showed that no hydroxy groups were present.

### Example 10 - Alternative fluorination of DEPEG 600

Fluorination of DEPEG 600 (prepared as in Procedure C) was carried out in the same manner as Example 8 except that in this case the weights of reagents used were as follows:

| | |
|---|---|
| DEPEG 600 | 4.5 g |
| acetone | 7.9 g |
| HFP | 15.5 g |

In this case 0.7 g HFP was recovered. Analysis of the product of formula (8) obtained showed that 14 molar equivalents of HFP had been incorporated.

### Example 11: Reaction of DEPEG 600 with octafluorocyclopentene

A Carius tube was charged with DEPEG 600 (1.1g, 1.7mmol), acetone (4ml) and octafluorocyclopentene (7.2g, 34mmol) and was irradiated with UV radiation in the manner described in Procedure A above. After irradiation fluoroalkene (0.6g) was recovered, and the acetone removed under reduced pressure. A dark brown, viscous liquid was obtained for which ¹H and ¹⁹F n.m.r spectroscopy indicated a composition of DEPEG 600 containing an average of five (2H-octafluorocyclopentyl) groups attached (formula (8), where R_{F}=2H-octafluorocyclopentyl). Higher molecular weight material was also obtained.

### Example 12: Reaction of DEPEG 600 with hexafluorocyclobutene

A Carius tube was charged with DEPEG 600 (1.3g, 2.0mmol), acetone (4ml) and hexafluorocyclobutene (11.6g, 71.6mmol). After UV irradiation as in Procedure A fluoroalkene (5.9g) was recovered, and the acetone was removed under reduced pressure. A dark brown, viscous liquid was obtained for which ¹H and ¹⁹F n.m.r. analysis indicated a compound of formula (8) having an average of five (2H-hexafluorocyclobutyl) groups attached. Higher molecular weight material was also obtained.

### Example 13: Reaction of PEG 600 with HFP

In the manner described in Example 7 a Carius tube was charged with poly(ethylene glycol) 600 (ie, material with an average molecular weight of 600) (3.2g 5.3mmol), acetone (15ml), and HFP (19.4g 129mmol) and irradiated with UV radiation as in Example 7. After reaction HFP (15.9g) was recovered, and acetone was removed under vacuum.
The ¹H and ¹⁹F n.m.r. spectra showed approximately four molar equivalents of HFP to have been incorporated into the poly(ethylene glycol). The product was of the general formula (20) as follows: 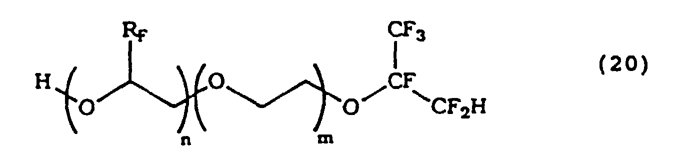 where R_{F} = CF₂CFHCF₃.

About half the hydroxy groups were found to have reacted with HFP in a nucleophilic manner (implying three molar equivalents of HFP to have reacted in a free radical manner and one equivalent nucleophilically). A viscous, brown material was obtained.

### Example 14: Reaction of diethylpoly(tetrahydrofuran) with HFP

In the manner described in Procedure A a Carius tube was charged with diethylpoly(tetrahydrofuran) (3.0g, 2.9 mmol) (ie, poly(tetrahydrofuran) of average molecular weight 1000 which had subsequently been diethylated via the process described below), acetone (19.0 g) and HFP (16.1g). After UV irradiation in the manner described in Procedure A excess HFP was recovered (5.7g), and acetone removed under vacuum. NMR studies showed an average of 26 molar equivalents of HFP had been incorporated in the pale brown liquid obtained (12.8g).
The product was of general formula (21) as follows: 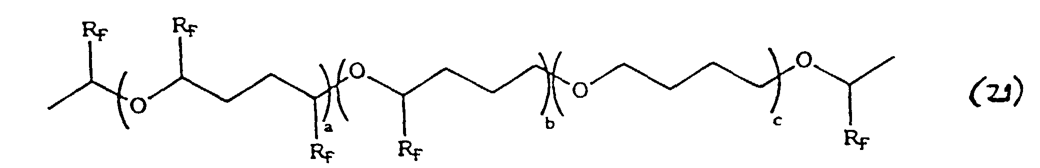 wherein R_{F} = CF₂CFCF₃.
and 2a + b = 24, a + b + c = 13

The diethylpoly(tetrahydrofuran) used in Example 14 was prepared by Procedure E as follows:

### Procedure E

Using the same method as in Procedure B described above, a dried solution of poly(tetrahydrofuran) of average molecular weight 1000 (46.9 g, 47 mmol) in toluene (90 ml) was dripped onto washed sodium hydride (4.5 g of 60% dispersion in oil, 110 mmol) and stirred (5 hours). Diethyl sulphate (14.7 g, 95 mmol) was subsequently added and stirring continued (8 hours). After removal of the volatiles, this was passed down an alumina column in dichloromethane and the solvent was removed. A white solid was obtained, α-ethyl-w-ethoxy poly(tetramethylene oxide) 1000 (DEPTHF) (27.0 g, 54.8%); (Found: C, 66.8; H, 11.3; C₅₆H₁₁₄O₁₄ requires C, 66.5; H, 11,4%); IR number 2; NMR number 3.

### Example 15 - Addition of 18-crown-6 polyether to HFP

A Carius tube, charged with 18-crown-6 polyether (4.2 g, 16 mmol), acetone (11.9 g) and HFP (17.9 g, 120 mmol), was irradiated with UV rays for 3 days as in Procedure A. HFP (0.4 g) was recovered and acetone removed under reduced pressure to leave a viscous, light brown liquid, poly(2H-hexafluoropropyl)-18-crown-6 formula (18), and NMR data indicated that 5 molar equivalents of HFP had been incorporated (11.6 g, 71.9%); (Found C, 32.3; H, 2.5: C₂₇H₂₄F₃₀O₆ requires C, 32.0; H, 2.4%); IR and NMR analysis were consistent with the structure specified.

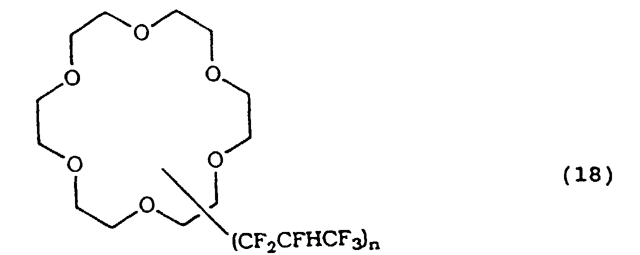

### Example 16 - Addition of 18-Crown-6 polyether to pentafluoropropene

A Carius tube was charged with 18-crown-6 polyether (1.5 g, 6 mmol), pentafluoropropene (5.5 g, 42 mmol) and acetone (10 ml) and irradiated with UV radiation as in Procedure A and Example 7. After UV irradiation 0.1 g of pentafluoropropene was recovered. Acetone was removed under vacuum, and a viscous, pale brown liquid was obtained which ¹H and ¹⁹F n.m.r. indicated was 18-crown-6 polyether to which an average of 5.0 (1',1',3',3',3'-pentafluoropropyl) groups had been added to give a compound of formula (22) as follows where n=5.

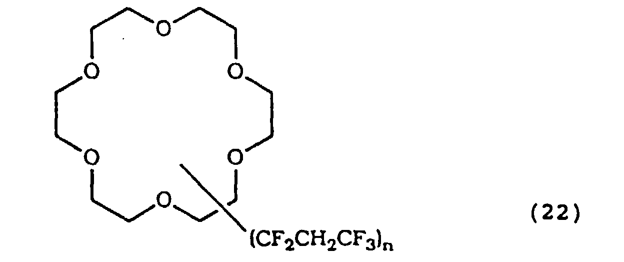

In the above mentioned comparative route using gamma-rays referred to in connection with Table 1 the following general procedure, Procedure F was used.

### Procedure F

Solid and liquid reagents were introduced into a Pyrex Carius tube (capacity 60 ml) together with solvent if used, and degassed twice by freeze-thawing. HFP was then introduced into the cooled (liquid air) Carius tube using standard vacuum-line techniques, the tube then being sealed, and placed in a metal sleeve. After regaining room temperature, the Carius tube was irradiated with gamma-rays from a ⁶⁰Co source (85 krad hr⁻¹). Subsequently, the tube was cooled (liquid air) and opened. Any remaining HFP was recovered as the tube returned to room temperature. Products were poured out.

### Comparative Example A1:

### Addition of DEPEG 600 to HFP using gamma-initiation.

A Carius tube was charged with DEPEG 600 (3.7 g, 5.7 mmol), acetone (9.9 g) and HFP (39.7 g, 265 mmol), and then irradiated with gamma-rays for 4 days as in Procedure F. HFP (34.2 g) was recovered, and acetone removed under vacuum, to leave a pale brown, viscous liquid (9.2 g), poly(2H-hexafluoropropyl)-α-ethyl-w-ethoxy- poly(ethylene oxide) 600, with 6 equivalents of HFP incorporated; (Found: C, 35.85; H 3.5; F, 46.2. C₅₁H₆₂F₄₂O₁₄ requires C, 36.0; H, 3.7; F, 47.0%); IR and NMR data were comparable to that of higher adducts.

### Comparative Example A2:

### Addition of DEPEG 600 to HFP using gamma-initiation.

A Carius tube was charged with DEPEG 2000 (4.7 g, 2.3 mmol), acetone (13.2 g) and HFP (30.3 g, 202 mmol), and then irradiated with gamma-rays for 7 days. HFP (23.1 g) was recovered, and acetone removed under vacuum, to leave a pale brown, viscous liquid (8.6 g),
poly(2H-hexafluoropropyl)-α-ethyl-w-ethoxy poly(ethylene oxide) 2000, with 14 equivalents of HFP incorporated; IR and NMR data were consistent with that of lower molecular weight samples; IR number 4.

## Claims

1. A process for the preparation of a perfluoropolyether having a general formula: 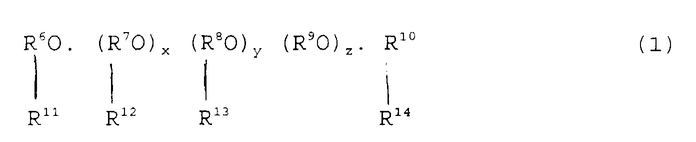 wherein the symbols R⁶ to R¹⁴ each represents a fully fluorinated hydrocarbon group, x is an integer and each of y and z is zero or an integer, and the sum of x, y and z is at least 2 and not greater than 100,
which process includes direct fluorination with elemental fluorine of a polyether, the polyether having a general formula: 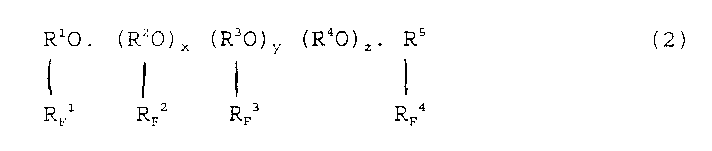 wherein the symbols R¹, R², R³, R⁴ and R⁵ each represent substituted or unsubstituted hydrocarbon groups, which groups may differ, the symbols R_{F}¹, R_{F}², R_{F}³ and R_{F}⁴ each represent fluorine-containing hydrocarbon groups containing at least two carbon atoms and at least two fluorine atoms, which groups may differ, or the combination R¹ - R_{F}¹ and/or the combination R⁵ - R_{F}⁴ represents a fluorine-containing alkyl or alkenyl group, wherein x, y, z have the same meaning as above, or cyclic polyethers; or the polyether being selected from diethyl (polyethylene oxides) ; dihydroxy (polyethylene oxides); diethyl (polytetrahydrofurans) and cyclic polyethers, characterised in that the fluorination is carried out in the presence of a solvent which is an ether having at least one substituent R_{F} group, wherein R_{F} is a fluorinated hydrocarbon group, the fluorination of the polyether proceeding in concert with the fluorination of the solvent.

2. A process as claimed in Claim 1 and wherein the said solvent ether is a fluorinated volatile ether.

3. A process as claimed in Claim 1 or Claim 2 and wherein the said solvent ether has at least two groups R_{F} which may be the same or different groups.

4. A process as claimed in Claim 3 and wherein each group R_{F} has from two to four carbon atoms and at least two fluorine atoms.

5. A process as claimed in any one of the preceding Claims and wherein the said solvent ether is an alicyclic ether having from 3 to 5 carbon atoms in its alicyclic ring.

6. A process as claimed in Claim 5 and wherein the said solvent ether is one of the formula:

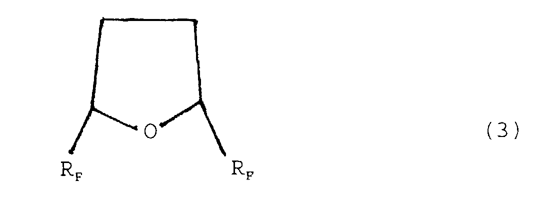

7. A process as claimed in any one of Claims 3 to 6 and wherein each R_{F} group is CF₂CFHCF₃.

8. A process according to any of the preceding Claims in which a gaseous mixture of fluorine and nitrogen is introduced into the substituted polyether in liquid form.

9. A process as in any one of Claims 1 to 8 and wherein the step of preparing the intermediates having units of general formula (2) as defined in Claim 1 includes the step of reacting a linear or branched chained or cyclic polyether of or having units of general formula: 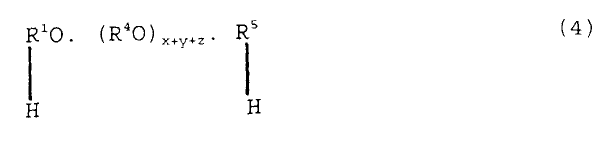 or 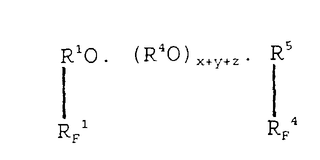 where R¹ and/or R⁵ each contain a single carbon atom and/or at least R⁴ contains at least 2 carbon atoms with a linear or branched chained or cyclic fluoroalkene containing at least two fluorine atoms in the presence of a sensitising medium which when energised helps to induce the reaction, ultra-violet radiation being applied to the said medium to provide energisation of the medium, wherein the said sensitising medium is an organic liquid which is a solvent for the reagents having a strong extinction coefficient in the spectral region of the applied ultra-violet radiation.

10. A process as in Claim 9 and wherein the said sensitising medium comprises acetone or trifluorethanol.

11. A process according to any preceding claim in which the cyclic polyethers are crown ethers.

12. A process as in any one of Claims 9 or 10 and wherein the fluoroalkene is selected from one of the following having five or more fluorine atoms fluoropropenes, fluorobutenes, fluoropentenes and fluorohexenes, fluorocyclobutenes, fluorocyclopentenes and fluorocyclohexenes.

13. A process according to any of claims 9 to 12 in which the radiation source has a power output of 0.1kw to 10kw.

14. A process according to any of claims 1 to 13 in which the process is carried out at a temperature of 50°C or below.

## Patentansprüche

1. Verfahren zur Herstellung eines Perfluorpolyether mit der allgemeinen Formel: 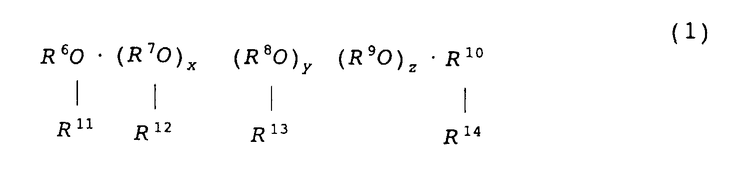 bei denen die Symbole R⁶ bis R¹⁴ je eine vollständige flouronierte Hydrocarbongruppe darstellt, x eine ganze Zahl ist und sowohl y als auch z Null oder eine ganze Zahl darstellen und bei der die Summe x, y und z wenigstens 2 und nicht größer als 100 ist,
wobei dieser Prozeß die direkte Fluorierung mit elementarem Fluor eines Polyethers umfaßt, wobei der Polyether die allgemeine Formel hat: 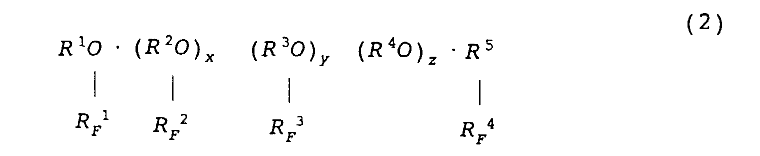 in der die Symbole R¹, R², R³, R⁴ und R⁵ je eine substituierte oder unsubstituierte Hydrocarbongruppe darstellt, wobei diese Gruppen unterschiedlich sein können, die Symbole R_{F}¹, R_{F}², R_{F}³ und R_{F}⁴ je eine Fluor enthaltene Hydrocarbongruppe darstellt, mit wenigstens zwei Kohlenstoffatomen und wenigstens zwei Fluoratomen, wobei die Gruppen unterschiedlich sein können oder eine Kombination R¹ - R_{F}¹ und/oder die Kombination R⁵ - R_{F}⁴ eine Fluor enthaltende Alkyl- oder Alkenegruppe repräsentiert, in der x, y, z die gleiche Bedeutung wie oben aufweisen oder cyclische Polyether; oder der Polyether ist ausgewählt von Diethyl (Polyethylenoxide); Dihydroxy (Polyethylenoxide); Diethyl (Polytetrahydrofurane) und cyclische Polyether,
dadurch gekennzeichnet,
daß die Fluorierung in der Anwesenheit eines Lösungsmittels ausgeführt wird, welches ein Ether ist, der wenigstens eine substituierte R_{F}-Gruppe aufweist, wobei R_{F} eine fluorisierte Hydrocarbongruppe darstellt, wobei die Fluorierung des Polyethers einhergeht im Einklang mit der Fluorierung des Lösungsmittels.

2. Verfahren nach Anspruch 1, bei dem der Lösungsmittelether ein fluorisierter, flüchtiger Ether ist.

3. Verfahren nach Anspruch 1 oder 2, bei dem der genannte Lösungsmittelether wenigstens zwei Gruppen R_{F} aufweist, die die gleiche oder unterschiedliche Gruppen sein können.

4. Verfahren nach Anspruch 3, bei dem jede Gruppe R_{F} zwei bis vier Kohlenstoffatome aufweist und zwei Fluoratome.

5. Verfahren nach einem der vorangehenden Ansprüche, bei dem der genannte Lösungsmittelether einen alicyclischen Ether darstellt mit drei bis fünf Kohlenstoffatomen in seinem alicyclischen Ring.

6. Verfahren nach Anspruch 5, bei dem der Lösungsmittelther einer nach der folgenden Formel ist:

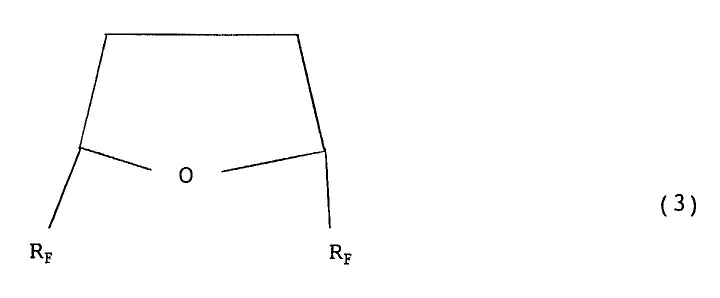

7. Verfahren nach einem der Ansprüche 3 bis 6, bei dem jede Gruppe R_{F} Gruppe CF₂CFHCF₃ ist.

8. Verfahren nach einem der vorangehenden Ansprüche, bei dem eine gasförmige Mischung von Fluor und Stickstoff in das substituierte Polyether in flüssiger Form eingeführt wird.

9. Verfahren nach einem der Ansprüche 1 bis 8, bei dem die Schritte der Herstellung der Zwischenstoffe Einheiten der allgemeinen Formel (2) aufweisen, wie in Anspruch 1 definiert, welches den Schritt der Reaktion eines linearen oder verzweigt verketteten oder cyclischen Polyethers oder Einheiten der allgemeinen Formel: 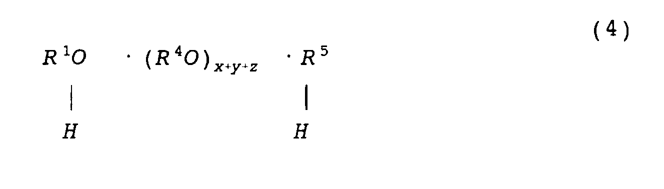 oder 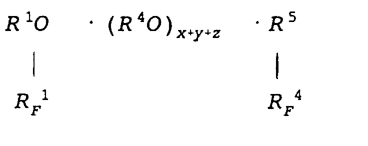 wobei R¹ und/oder R⁵ je ein einfaches Kohlenstoffatom und/ oder wenigstens R⁴ wenigstens zwei Kohlenstoffatome enthält mit einem linearen oder verzweigt verketteten oder cyclischen Fluoralken, mit wenigstens zwei Fluoratomen in der Anwesenheit eines lichtempfindlichen Mediums, welches, wenn es aktiviert wird, hilft, die Reaktion zu induzieren, wobei ultraviolette Strahlung auf das genannte Medium aufgegeben wird zur Energiesierung des Mediums, wobei das genannte sensitive, lichtempfindliche Medium eine organische Flüssigkeit ist, die ein Lösungsmittel für die Reagensien darstellt mit einem starken Auslöschkoeffizienten in der Spektralregion der aufgelegten ultravioletten Strahlung.

10. Verfahren nach Anspruch 9, bei dem das lichtempfindliche Medium Aceton oder Trifluorethanol umfaßt.

11. Verfahren nach einem der vorangehenden Ansprüche, bei dem die cyclischen Polyether Kronenether darstellen.

12. Verfahren nach einem der Ansprüche 9 oder 10, bei dem das Fluoralken von einer der folgenden, fünf oder mehr Fluoratome enthaltenden Fluoropropen, Fluorobuten, Fluoropenten und Fluorohexan, Fluorocyclobuten, Fluorocyclopentan und Fluorocyclohexan ausgewählt wird.

13. Verfahren nach einem der Ansprüche 9 bis 12, in dem die Strahlungsquelle eine Leistung von ca. 0,1 kW bis 10 kW aufweist.

14. Verfahren nach einem der Ansprüche 1 bis 13, bei dem der Prozeß bei einer Temperatur von 50°C oder niedriger ausgeführt wird.

## Revendications

1. Procédé pour la préparation d'un perfluoropolyéther ayant une formule générale: 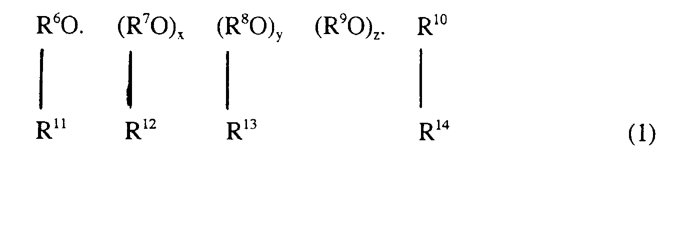 dans laquelle les symboles R⁶ à R¹⁴ représentent chacun un groupe hydrocarbure complètement fluoré, x est un nombre entier et chacun de y et z est zéro ou un nombre entier, et la somme de x, y et z est d'au moins 2 et non supérieure à 100, ce procédé incluant la fluoration directe avec du fluor élémentaire d'un polyéther, le polyéther ayant une formule générale : 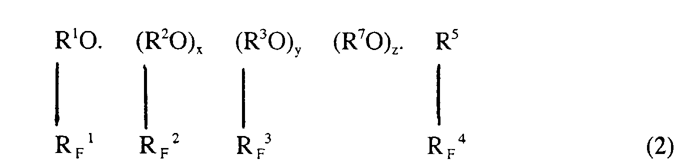 dans laquelle les symboles R¹, R², R³, R⁴ et R⁵ représentent chacun des groupes hydrocarbures substitués ou non substitués, ces groupes pouvant varier, les symboles R_{F}¹, R_{F}², R_{F}³ et R_{F}⁴ représentent chacun des groupes hydrocarbures contenant du fluor contenant au moins deux atomes de carbone et au moins deux atomes de fluor, ces groupes pouvant varier, ou la combinaison R¹-R_{F}¹ et/ou la combinaison R⁵-R_{F}⁴ représente un groupe alcényle ou alkyle contenant du fluor, dans laquelle x, y et z ont la même signification que ci-dessus, ou des polyéthers cycliques ; ou le polyéther étant choisi parmi des polyéthylène oxydes diéthyliques ; des dihydroxy(polyéthylène oxydes) ; des polytétrahydrofurannes diéthyliques et des polyéthers cycliques, caractérisé en ce que la fluoration est effectuée en présence d'un solvant qui est un éther ayant au moins un groupe R_{F} substituant, dans lequel R_{F} est un groupe hydrocarbure fluoré, la fluoration du polyéther se poursuivant de concert avec la fluoration du solvant.

2. Procédé tel que revendiqué dans la revendication 1 et dans lequel ledit éther solvant est un éther volatile fluoré.

3. Procédé tel que revendiqué dans la revendication 1 ou la revendication 2 et dans lequel ledit éther solvant possède au moins deux groupes R_{F} qui peuvent être les mêmes groupes ou des groupes différents.

4. Procédé tel que revendiqué dans la revendication 3 et dans lequel chaque groupe R_{F} possède de deux à quatre atomes de carbone et au moins deux atomes de fluor.

5. Procédé tel que revendiqué dans l'une quelconque des revendications précédentes et dans lequel ledit éther solvant est un éther alicyclique ayant de 3 à 5 atomes de carbone dans son cycle alicyclique.

6. Procédé tel que revendiqué dans la revendication 5 et dans lequel ledit éther solvant est un de formule :

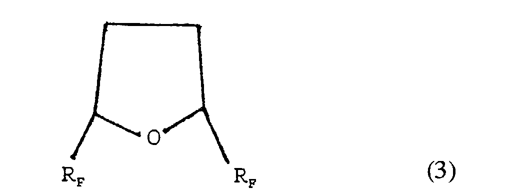

7. Procédé tel que revendiqué dans l'une quelconque des revendications 3 à 6 et dans lequel chaque groupe R_{F} est CF₂CFHCF₃.

8. Procédé selon l'une quelconque des revendications précédentes, dans lequel un mélange gazeux de fluor et d'azote est introduit dans le polyéther substitué sous forme liquide.

9. Procédé tel que dans l'une quelconque des revendications 1 à 8 et dans lequel l'étape de préparation des intermédiaires ayant des motifs de formule générale (2) telle que définie dans la revendication 1, inclut l'étape consistant à faire réagir un polyéther à chaîne linéaire ou ramifiée ou cyclique de formule générale ou ayant des motifs de formule générale : 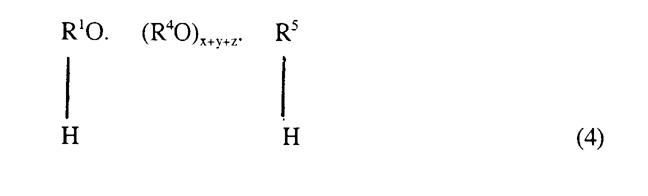 ou 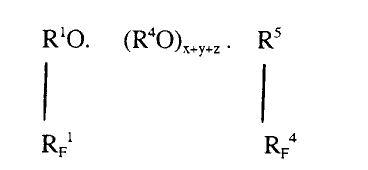 où R¹ et/ou R⁵ contiennent chacun un unique atome de carbone et/ou au moins R⁴ contient au moins 2 atomes de carbone, avec un fluoroalcène à chaîne linéaire ou ramifiée ou cyclique contenant au moins deux atomes de fluor, en présence d'un milieu sensibilisant qui lorsqu'il est activé aide à induire la réaction, un rayonnement ultraviolet étant appliqué audit milieu pour fournir l'activation du milieu, dans lequel ledit milieu sensibilisant est un liquide organique qui est un solvant pour les réactifs ayant un fort coefficient d'extinction dans la région spectrale du rayonnement ultraviolet appliqué.

10. Procédé tel que dans la revendication 9 et dans lequel ledit milieu sensibilisant comprend de l'acétone ou du trifluoréthanol.

11. Procédé selon l'une quelconque des revendications précédentes dans lequel les polyéthers cycliques sont des éthers couronnes.

12. Procédé tel que dans l'une quelconque des revendications 9 ou 10 et dans lequel le fluoroalcène est choisi parmi l'un des fluoropropènes, des fluorobutènes, des fluoropentènes et des fluorohexènes, des fluorocyclobutènes, des fluorocyclopentènes et des fluorocyclohexènes ayant cinq atomes de fluor ou plus.

13. Procédé selon l'une quelconque des revendications 9 à 12, dans lequel la source de rayonnement possède une puissance de sortie de 0,1 kW à 10 kW.

14. Procédé selon l'une quelconque des revendications 1 à 13, dans lequel le procédé est effectué à une température de 50°C ou moins.
